# EUROPÄISCHE PATENTSCHRIFT

(11) **EP 1 569 979 B1**
(45) Veröffentlichungstag und Bekanntmachung des Hinweises auf die Patenterteilung: **22.02.2012**
(21) Anmeldenummer: 03782135.2
(22) Anmeldetag: 06.12.2003
(51) Int. Cl.: C08G 18/67

(54) **VERWENDUNG EINES WERKSTOFFES FÜR LÄRM-, SCHWIMMSCHÜTZER, OTOPLASTIKEN UND ZUR FIXIERUNG VON VENTINGS SOWIE VERFAHREN ZUR HERSTELLUNG SOLCHER TEILE**
USE OF A MATERIAL FOR NOISE PROTECTION DEVICES, SWIMMING PROTECTION DEVICES, OTOPLASTICS AND FOR SECURING VENTINGS AND METHOD FOR THE PRODUCTION OF SAID PARTS
UTILISATION D'UNE MATIERE POUR DES DISPOSITIFS DE PROTECTION AUDITIVE ET DE PROTECTION AURICULAIRE POUR LES NAGEURS, POUR DES EMBOUTS AURICULAIRES ET POUR LA FIXATION DE DISPOSITIFS DE DECHARGE AURICULAIRE, AINSI QUE PROCEDE DE FABRICATION DE CES PIECES

(30) Priorität: 12.12.2002 DE 10258361
(43) Veröffentlichungstag der Anmeldung: 07.09.2005
(73) Patentinhaber: Dreve Otoplastik GmbH, 59423 Unna (DE)
(72) Erfinder: KLARE, Martin, 44137 Dortmund (DE); ALTMANN, Reiner, 44575 Castrop-Rauxel (DE); KUTSCHINSKI, Michael, 44579 Castrop-Rauxel (DE); VEIT, Thomas, 48151 Münster (DE); MEISSNER, Georgia, 44263 Dortmund (DE)
(74) Vertreter: Köchling, Conrad-Joachim
(86) Internationale Anmeldenummer: PCT/DE2003/004016
(87) Internationale Veröffentlichungsnummer: WO 2004/052957

(56) Entgegenhaltungen:
- EP-A- 0 498 592
- WO-A-96/15179
- DE-A- 4 107 284
- US-A- 5 763 503

## Beschreibung

Die vorliegende Erfindung betrifft eine Verwendung eines Werkstoffes für Lärm-, Schwimmschützern, Otoplastiken und zur Fixierung von Ventings, sowie ein Verfahren zu deren Herstellung.

Zur Herstellung von Lärm-, Schwimmschützern und Otoplastiken kommen gegenwärtig sowohl indirekte als auch direkte Verfahren zu Einsatz. Die Anforderungen an den Tragekomfort dieser Maß-Ohr-Passstücke sind nur zu erreichen, wenn das gesamte Herstellungsverfahren auf absolute Formtreue und Passgenauigkeit zielend optimiert ist. Die indirekten Verfahren bestehen aus dem Anfertigen eines Abdruckes, eines Negativs des Abdruckes und dem anschließenden Rohlingguss. Jeder der Einzelschritte ist mit einer Reihe von Fehlermöglichkeiten behaftet, die zu einer fehlerhaften Abformung führen können. Aus diesen Gründen ist es wünschenswert, das Ohrpassstück aus der Original-Abformung des Ohres direkt zu fertigen. Die Direktmethode stellt so die einfachste und auch die wirtschaftlichste Art der Ohrstückfertigung dar. Als nachteilig ist für die direkten Verfahren jedoch zu bemerken, dass die Materialauswahl im Wesentlichen auf RTV-Silikon-Kautschuke beschränkt ist und sich so keine Im-Ohr-Geräteschalen direkt im Ohr herstellen lassen.

Der Erfindung liegt die Aufgabe zugrunde, eine Verwendung eines Werkstoffes für Lärm-, Schwimmschützer und Otoplastiken sowie ein Verfahren zu deren Herstellung zur Verfügung zu stellen, die bzw. das eine passgenaue und einfache Herstellung und Nachbearbeitung ermöglichen.

Diese Aufgabe wird durch die Verwendung von Materialien für Lärm-, Schwimmschützer und Otoplastiken gelöst, die eine zweistufige Härtung des Materials durch Diisocyanat-Polyaddition und radikalische Polymerisation ermöglichen und mindestens ein Diisocyanat, wie z.B. Hexamethylendiisocyanat (HDI), Isophorondiisocyanat (IPDI) oder Bis-(4-isocyanatocyclohexyl)- Methan (H₁₂ MDI), sowohl in monomerer als auch in oligomerer Form, beispielsweise in Form von Trimeren, und mindestens eine Hydroxylverbindung mit mindestens zwei OH-Gruppen enthalten. Die erfindungsgemäß verwendeten Materialien für Lärm-, Schwimmschützer und Otoplastiken enthalten vorzugsweise mindestens 5-60 Gew.-%, besonders bevorzugt 7,5-45 Gew.-% und ganz besonders bevorzugt 9-20 Gew.-% des Diisocyanates, bezogen auf die Gesamtmasse des Materials.

Als weitere Komponente enthalten die erfindungsgemäß verwendeten Materialien für Lärm-, Schwimmschützer und Otoplastiken mindestens eine Hydroxylverbindung. Diese bildet mit dem Isocyanat durch Polyaddition ein Polyurethan.

Unter den Hydroxylverbindungen sind Polyole, unter denen Verbindungen mit mindestens 3 Hydroxylgruppen verstanden werden, als besonders geeignet hervorzuheben. Als Hydroxylgruppen sind hier alkoholische OH-Gruppen gemeint. Dabei sind monomere oder polymere aliphatische, alicyclische oder aromatische Verbindungen mit 3-8 OH-Gruppen insbesondere bevorzugt. Darüber hinaus können auch aliphatische und cycloaliphatische Diole verwendet werden, wie beispielsweise Ethylenglykol, Di- und Triethylenglykol, 1,2- und 1,3- Propandiol, Di- und Tripropylenglykol, 1,2- und 1,3- oder 1,4-Butandiol, 1,6-Hexandiol und Cyclohexandiol. Unter den polymeren Polyolen eignen sich besonders Polyesterpolyole, Polyetherpolyole und Polyalkohole, die sowohl Ether- als auch Estergruppen enthalten. Dabei sind solche Hydroxylverbindungen besonders bevorzugt, die ein Äquivalentgewicht von 50 bis 1000 pro Hydroxylgruppe aufweisen. Weiterhin sind solche Verbindungen bevorzugt, die eine verzweigte Struktur aufweisen.

Zur Herstellung der Materialien zur Verwendung für Lärm-, Schwimmschützer und Otoplastiken sind als besonders geeignet Hydroxylverbindungen zu nennen, die neben den Hydroxylgruppen radikalisch polymerisierbare Gruppen wie Vinyl-, Acryl und/oder Methacrylgruppen aufweisen. So kann eine zusätzliche Vernetzung der durch die Polyaddition gebildeten Polyurethanketten mittels radikalischer Polymerisation erfolgen. Zur Gruppe der bevorzugten Hydroxylverbindungen mit den o.g. radikalisch polymerisierbaren Gruppen sind beispielsweise Hydroxyethyl(meth)-acrylate wie 2-Hydroxyethylacrylat, 2-Hydroxyethylmethacrylat, 1,4-Butandiolacrylat, 1,4-Butandioldimethacrylat, 2-Hydroxypropyl-acrylat, 2-Hydroxypropylmethacrylat, 4-Hydroxybutylacrylat und 4-Hydroxybutylmethacrylat zu zählen.

Als Hydroxylkomponenten können vorteilhaft auch Mischungen der o.g. Hydroxylverbindungen verwendet werden. Dabei sind Mischungen aus monomeren und polymeren Hydroxylverbindungen bevorzugt.

Die Konzentration an Hydroxylverbindungen in den Materialien für Lärm-, Schwimmschutz und Otoplastiken ist abhängig von der Menge der Isocyanatkomponente und liegt bevorzugt im Bereich von 4-40 Gew.-%, bezogen auf die Gesamtmasse des Materials.

Die verwendeten Materialien für Lärm-, Schwimmschützer und Otoplastiken, die neben der Isocyanatkomponente radikalisch polymerisierbare Hydroxylverbindungen enthalten, können in zwei Stufen ausgehärtet werden. Der Werkstoff kann zunächst in teigiger Form in den Gehörgang des Patienten eingebracht werden und wird dort zunächst vorgehärtet. Der Formling kann dann entnommen und im angehärteten Zustand modelliert, bearbeitet und angepasst werden, bevor dann durch Strahlung endgehärtet wird. Dabei wird in der ersten Stufe das Material durch die Isocyanat-Polyaddition vorgehärtet und besitzt in diesem Zustand bereits Festigkeit und Formbeständigkeit die es ermöglicht, dass der Werkstoff noch aus dem Ohr entfernt und überschüssiges Material leicht abgetragen werden kann. So können beispielsweise bei Otoplastiken auf einfache Art Hohlräume für die einzubauende Elektronik geschaffen werden. Die Endhärtung erfolgt dann durch radikalische Polymerisation.

Neben den Hydroxylverbindungen enthalten die Materialien für Lärm-, Schwimmschutz und Otoplastiken in einer bevorzugten Ausführungsform auch ein oder mehrere radikalisch polymerisierbare Monomere. Diese können gegebenenfalls mit den Hydroxylverbindungen copolymerisieren, die radikalisch polymerisierbare Gruppen besitzen. Im Rahmen der Erfindung sind Materialien als besonders geeignet einzustufen, die sowohl radikalisch polymerisierbares Monomer bzw. Monomere und radikalisch polymerisierbare Hydroxylverbindungen aufweisen.

Unter den radikalisch polymerisierbaren Monomeren eignen sich besonders mono- und polyfunktionelle (Meth)acrylate wie 2,2-Bis[4-(acryloyloxy-ethoxy)phenyl]propan, 2,2-Bis [4-(2-hydroxy-3-acryloyloxypropan)phenyl]propan, 1,3 Butandioldi(meth)acrylat, 1,4-Butandioldi(meth)acrylat, 1,6-Hexandioldi(meth)acrylat, Mono-, Di-, Tri- und Tetraethylenglykoldi(meth)acrylate, Isobornylacrylat, 2-10fach ethoxilierte Bisphenol-A-Di(meth)acrylate. Als Monomere eignen sich auch Urethandi(meth)acrylate wie z.B. 7,7,9-Trimethyl-4,13-dioxo-3,14-dioxa-5,12-diaza-hexadecan-1,16-diol-dimethacrylat bzw. 7,9,9- Trimethyl-4,13-dioxo-3,14-dioxa-5,12-diaza-hexadecan-1,16-diol-dimethacrylat. Unter denjenigen Monomeren und ebenso Monomermischungen der oben aufgeführten Verbindungen sind diejenigen besonders bevorzugt, die mindestens ein Monomer mit 2 oder mehr radikalisch polymerisierbaren Gruppen enthalten und so als Vernetzer wirken können.

Die erfindungsgemäß verwendeten Materialien für Lärm-, Schwimmschutz und Otoplastiken enthalten in einer bevorzugten Ausführungsform auch Füllstoffe. Es können praktisch alle bekannten Füllstoffe wie z.B. anorganische Glas-, Glaskeramik- und Keramikpulver, pyrogene und gefällte Kieselsäuren, Zeolithe, gefüllte und ungefüllte organische Füllstoffe auf Basis von Poly(meth)acrylaten oder Polycarbonaten sowie Mischungen der genannten Füller eingesetzt werden. Die Größe der Füllstoffe sollte bevorzugt im Bereich von 10nm bis 200µm und besonders bevorzugt im Bereich zwischen 12nm und 50µm liegen.

Als Initiatoren für die radikalische Polymerisation enthalten die erfindungsmäßigen Materialien für Lärm-, Schwimmschutz und Otoplastiken bevorzugt Initiatoren wie Campherchinon, 2,4,6-Trimethylbenzoylphenylphosphinoxid, Bis(2,4,6-trimethylbenzoyl)-phenylphophinoxid, 2,4,6-Trimethylbenzoyldiphenyl-phoshinoxid, 2-Methyl-1,4-(methylthio)phenyl-2-morpholinopropan-1-on, Bis(2,6-dimethoxybenzoyl)-2,4,4-trimethylpentylphosphinoxid, 2-Hydroxy-2-methyl-1-phenyl-propan-1-on, 2-Benzyl-2-dimethylamino-1-(4-morpholinophenyl)-butanon-1, 2,2-Dimethoxy-1,2-diphenylethan-1-on, 1-Hydroxy-cyclohexyl-phenylketon, die in der US 4,746,686 beschriebenen und Mischungen der o.g. Initiatoren. Darüber hinaus können auch Initiatoren wie z.B. Dibenzoylperoxid verwendet werden.

Die Initiatoren können bevorzugt in Kombination mit Synergisten aus der Verbindungsklasse der tertiären Amine wie z.B. N-Methyldiethanolamin, Dimethyl-p-toluidin, Dihydroxyethyl-p-toluidin oder Triethanolamin eingesetzt werden.

Weiterhin können in den erfindungsgemäß verwendeten Materialien für Lärm-, Schwimmschutz und Otoplastiken Stabilisatoren eingesetzt werden, um eine frühzeitige Polymerisation des Materials zu verhindern. Als Stabilisatoren können die bekannten und bewährten Verbindungen wie 2,6-Di-tert.-butyl-p-kresol, Hydrochinon, Hydrochinomonomethylether, Phenol und Benzochinon verwendet werden, wobei 2,6-Di-tert.-butysl-p-kresol bevorzugt ist. Als Katalysatoren für die Diisocyanat-Polyaddition können die bekannten und bewährten Zinnorganyle verwendet werden, wie z.B. Di-n-octylzinncarboxylate, Dibutylzinnoxid, Dioctylzinnoxid, Dibutylzinnglycolat, -dilaurat, - diacetat, -maleat, Dibutylzinnmaleinatester, Dilaurylzinndiacetat, Di-n-octyl-zinnmaleinatester, Dibutylzinnalkylmercaptide und Dibutylzinnmercaptoester.

Darüber hinaus können Additive wie Weichmacher (z.B. Dibutylphthalat) oder antimikrobielle Zusätze wie z.B. Triclosan und Chlorhexidin und Pigmente (z.B. TIO₂) zugegeben werden.

Die erfindungsgemäß verwendeten Materialien für Lärm-, Schwimmschützer und Otoplastiken werden in Form von zwei Komponenten bereitgestellt, so dass die reaktiven Komponenten des Materials, wie beispielsweise das Isocyanat und die Hydroxylverbindung, voneinander getrennt vorliegen. In einer bevorzugten Form enthält die erste Komponente die Hydroxylverbindung und die zweite Komponente die Isocyanatverbindung, so dass durch Mischen der beiden Komponenten die Polyurethanbildung ausgelöst werden kann. Dabei enthält die erste Komponente, auch als Basenpaste bezeichnet, nicht nur die Hydroxylverbindung sondern auch soweit vorhanden das radikalisch polymerisierbare Monomer, Füllstoffe, Katalysatoren, Stabilisatoren, Synergisten und Additive. Die zweite Komponente, auch als Katalysatorpaste bezeichnet, enthält soweit vorhanden neben dem Isocyanat auch den Füllstoff, Stabilisatoren und Additive. Die erfindungsgemäßen Materialien eignen sich vorzugsweise für die Herstellung von Lärm-, Schwimmschützer und Otoplastiken, können aber auch als so genanntes Ventinggel zur Befestigung von Ventings in Ohrstücken eingesetzt werden.

Die zweikomponentige Zusammensetzung der Materialien ist vorzugsweise so eingestellt, dass die Einzelkomponenten in Mischungsverhältnissen von 10:1 bis 1:1 (bezogen auf das Volumen) vorliegen. In einer bevorzugten Ausführungsform der Erfindung wird eine größere Menge der Basenpaste mit einer kleineren Menge an Katalysatorpaste umgesetzt.

### Beispiel:

Beispiel 1: Selbst- und lichthärtendes Material zur Herstellung von Lärm-, Schwimmschützern und Otoplastiken im Mischungsverhältnis von 4:1

### Basenpaste:

| Anteil, Gew.-% | Bestandteil |
|---|---|
| 0-60 Gew.% | Füllstoffe |
| 1-40 Gew.% | radikalisch polymerisierende Monomere |
| 1-50 Gew.% | Hydroxylkomponente |
| 0- 6 Gew.% | Synergist für die radikalische Polymerisation |
| 0- 5 Gew.% | Initiator für die radikalische Polymerisation |
| 0- 4 Gew.% | Zinnkatalysatoren |
| 0- 1 Gew.% | Pigmente |
| 0-15 Gew.% | Additive |

### Katalysatorenpaste:

| Anteil, Gew.-% | Bestandteil |
|---|---|
| 80-100 Gew.% | Diisocyanat |
| 0- 20 Gew.% | Füllstoffe |

## Patentansprüche

1. Verwendung eines Werkstoffes zur Herstellung von in das menschliche Ohr und/oder die Ohrmuschel einsetzbaren Lärmschutzformteilen, Schwimmschutzformteilen, Otoplastiken und Verwendung als Ventinggel zur Fixierung von Ventings in Ohrformstücken, wobei der Werkstoff aus mindestens einem Diisocyanat und mindestens einer Hydroxylverbindung mit mindestens zwei OH-Gruppen besteht, so dass eine Vorhärtung des Werkstoffes durch Diisocyanat-Polyaddition und eine Endhärtung durch radikalische Polymerisation erfolgt.

2. Verwendung nach Anspruch 1, **dadurch gekennzeichnet, dass** als Diisocyanat Hexamethylendiisocyanat (HDI), Isophorondiisocyanat (IPDI) oder Bis-(4-isocyanatocyclohexyl)- Methan (H₁₂ MDI) in monomerer oder in oligomerer Form, z.B. in Form von Trimeren vorgesehen ist.

3. Verwendung nach einem der Ansprüche 1 oder 2, **dadurch gekennzeichnet, dass** der Werkstoff mindestens 5 bis 60 Gew.%, vorzugsweise 7,5 bis 45 Gew.%, besonders bevorzugt 9 bis 20 Gew.% Diisocyanat, bezogen auf die Gesamtmasse des Werkstoffes, enthält.

4. Verwendung nach einem der Ansprüche 1 bis 3, **dadurch gekennzeichnet, dass** der Werkstoff als Hydroxylverbindung Polyole, nämlich Verbindungen mit mindestens drei Hydroxylgruppen vorgesehen sind.

5. Verwendung nach Anspruch 4, **dadurch gekennzeichnet, dass** als Hydroxylgruppen alkoholische OH-Gruppen eingesetzt sind.

6. Verwendung nach Anspruch 5, **dadurch gekennzeichnet, dass** monomere oder polymere aliphatische, alicylische oder aromatische Verbindungen mit drei bis acht OH-Gruppen eingesetzt sind.

7. Verwendung nach einem der Ansprüche 1 bis 3, **dadurch gekennzeichnet, dass** als Hydroxylverbindungen aliphatische und/oder cycloaliphatische Diole eingesetzt sind, insbesondere Ethylenglykol, Di- und Triethylenglykol, 1,2- und 1,3- Propandiol, Di- und Tripropylenglykol, 1,2- und 1-3- oder 1,4-Butandiol, 1,6- Hexandiol und Cyclohexandiol.

8. Verwendung nach einem der Ansprüche 4 bis 6, **dadurch gekennzeichnet, dass** als Hydroxylverbindungen Polyesterpolyole, Polyetherpolyole und/oder Polyalkohole, die sowohl Ether- als auch Estergruppen enthalten, eingesetzt sind.

9. Verwendung nach einem der Ansprüche 1 bis 8, **dadurch gekennzeichnet, dass** Hydroxylverbindungen mit einem Äquivalentgewicht von 50 bis 1000 pro Hydroxylgruppe eingesetzt sind.

10. Verwendung nach einem der Ansprüche 1 bis 9, **dadurch gekennzeichnet, dass** Hydroxylverbindungen eingesetzt sind, die eine verzweigte Struktur aufweisen.

11. Verwendung nach einem der Ansprüche 1 bis 10, **dadurch gekennzeichnet, dass** der Werkstoff außer den Hydroxylgruppen radikalisch polymerisierbare Gruppen wie Vinyl-, Acryl- und/oder Methacrylgruppen aufweist, vorzugsweise Hydroxyethyl(meth)-acrylate wie 2-Hydroxyethylacrylat, 2-Hydroxyethylmethacrylat, 1,4-Butandiolacrylat, 1,4-Butandioldimethacrylat, 2-Hydroxypropyalacrylat, 2-Hydroxypropylmethacrylat, 4-Hydroxybutylacrylat und 4-Hydroxybutylmethacrylat, sowie Mischungen daraus, insbesondere Mischungen aus monomeren und polymeren Hydroxylverbindungen enthält.

12. Verwendung nach Anspruch 1 bis 11, **dadurch gekennzeichnet, dass** die Konzentration der Hydroxylverbindungen im Bereich von 4 bis 40 Gew.% bezogen auf die Gesamtmasse des Werkstoffes beträgt.

13. Verwendung nach einem der Ansprüche 1 bis 12, **dadurch gekennzeichnet, dass** der Werkstoff radikalisch polymerisierbare Monomere enthält, insbesondere mono- und polyfunktionelle (Meth)acrylate, wie 2,2-Bis[4-(acryloyloxy-ethoxy)phenyl]propan, 2,2-Bis [4-(2-hydroxy-3-acryloyloxypropan)phenyl]propan, 1,3-Butandioldi(meth)acrylat, 1,4-Butandioldi(meth)acrylat, 1,6-Hexandioldi(meth)acrylat, Mono-, Di-, Tri- und Tetraethylenglykoldi (meth) acrylate, Isobornylacrylat, 2-10fach ethoxilierte Bisphenol-A-Di(meth)acrylate.

14. Verwendung nach Anspruch 13, **dadurch gekennzeichnet, dass** der Werkstoff Monomere enthält, nämlich Urethandi(meth)acrylate wie z.B. 7,7,9-Trimethyl-4,13-dioxo-3,14-dioxa-5,12-diaza-hexadecan-1,16-diol-dimethacrylat bzw. 7,9,9- Trimethyl-4,13-dioxo-3,14-dioxa-5,12-diaza-hexadecan-1,16-diol-dimethacrylat.

15. Verwendung nach einem der Ansprüche 13 oder 14, **dadurch gekennzeichnet, dass** der Werkstoff Monomere oder Monomermischungen enthält, die mindestens ein Monomer mit mindestens zwei radikalisch polymerisierbaren Gruppen aufweist und so als Vernetzer wirken.

16. Verwendung nach einem der Ansprüche 1 bis 15, **dadurch gekennzeichnet, dass** der Werkstoff Füllstoffe enthält, insbesondere anorganische Glas-, Glaskeramik- und Keramikpulver, pyrogene und gefällte Kieselsäuren, Zeolithe, gefüllte und ungefüllte organische Füllstoffe auf Basis von Poly(meth)acrylaten oder Polycarbonaten sowie Mischungen der genannten Füller.

17. Verwendung nach Anspruch 16, **dadurch gekennzeichnet, dass** die Größe der Füllstoffe im Bereich von 10 nm bis 200 µm liegt, vorzugsweise im Bereich zwischen 12 nm und 50 µm.

18. Verwendung nach einem der Ansprüche 1 bis 17, **dadurch gekennzeichnet, dass** der Werkstoff als Initiatoren für die radikalische Polymerisation Campherchinon, 2,4,6-Trimethylbenzoylphenylphosphinoxid, Bis(2,4,6-trimethylbenzoyl)-phenylphophinoxid, 2,4,6-Trimethylbenzoyldiphenyl-phoshinoxid, 2-Methyl-1,4-(methylthio)phenyl-2-morpholinopropan-1-on, Bis(2,6-dimethoxybenzol)-2,4,4-trimethylpentylphosphinoxid, 2-Hydroxy-2-methyl-1-phenyl-propan-1-on, 2-Benzyl-2-dimethylamino-1-(4-morpholinophenyl)-butanon-1, 2,2-Dimethoxy-1,2-diphenylethan-1-on, 1-Hydroxy-cyclohexyl-phenyl-keton, sowie Mischungen daraus oder auch Dibenzoylperoxid enthält.

19. Verwendung nach Anspruch 18, **dadurch gekennzeichnet, dass** die Initiatoren in Kombination mit Synergisten aus der Verbindungsklasse der tertiären Amine wie z.B. N-Methyldiethanolamin, Dimethyl-p-toluidin, Dihydroxyethyl-p-toluidin oder Triethanolamin eingesetzt sind.

20. Verwendung nach einem der Ansprüche 1 bis 19, **dadurch gekennzeichnet, dass** der Werkstoff Stabilisatoren enthält, um eine frühezeitige Polymerisation des Werkstoffes zu verhindern, insbesondere Verbindungen wie 2,6-Di-tert.-butyl-p-kresol, Hydrochinon, Hydrochinonmonomethylether, Phenol und Benzochinon wobei 2,6-Di-tert.-butyl-p-kresol bevorzugt ist.

21. verwendung nach einem der Ansprüche 1 bis 20, **dadurch gekennzeichnet, dass** der Werkstoff Katalysatoren für die Diisocyanat-Polyaddition in Form von Zinnorganyle enthält, insbesondere Di-n-octylzinncarboxylate, Dibutylzinnoxid, Dioctylzinnoxid, Dibutylzinnglycolat, - dilaurat, -diacetat, -maleat, Dibutylzinnmaleinatester, Dilaurylzinndiacetat, Di-n-octyl-zinnmaleinatester, Dibutylzinnalkylmercaptide und Dibutylzinnmercaptoester.

22. Verfahren zur Herstellung von Lärmschutzformteilen, Schwimmschutzformteilen oder Otoplastiken aus einem Werkstoff nach einem der Ansprüche 1-21, **dadurch gekennzeichnet, dass** der Werkstoff in zwei Komponenten bereitgestellt wird, so dass die reaktiven Komponenten des Materials nämlich das Diisocyanat und die Hydroxylverbindung voneinander getrennt vorliegen, dass die Komponenten gemischt werden und die Mischung in das Ohr oder die Ohrmuschel eingebracht wird und dort in einer ersten Verfahrensstufe das Material durch die Isocyanat-Polyaddition vorgehärtet wird, anschließend der Formling aus dem Ohr entnommen wird, gegebenenfalls nachbearbeitet wird und anschließend in einer zweiten Verfahrensstufe die Endhärtung durch radikalische Polymerisation durchgeführt wird.

23. Verfahren nach Anspruch 22, **dadurch gekennzeichnet, dass** die beiden Komponenten des Werkstoffes in Mischungsverhältnissen von 10:1 bis 1:1 -bezogen auf das Volumen- eingesetzt werden, vorzugsweise eine größere Menge an Hydroxylverbindung.

24. Verfahren nach einem der Ansprüche 22 oder 23, **dadurch gekennzeichnet, dass** die eine Komponente in Form einer Mischung aus der Hydroxylverbindung, radikalisch polymerisierbarem Monomer, Füllstoffen, Katalysatoren, Stabilisatoren, Synergisten und gegebenenfalls Additiven zur Verfügung gestellt wird sowie die zweite Komponente in Form einer Mischung aus Diisocyanat, Füllstoff, Stabilisatoren und gegebenenfalls Additiven.

## Claims

1. The use of a material for the production of noise protection parts, swimming protection parts, otoplastics to be inserted into the human ear and/or the auricle and for the use as a venting gel for securing ventings in ear impression parts, wherein said material is made of at least one diisocyanate and at least one hydroxyl compound comprising at least two OH groups, such that pre-hardening of the material occurs by means of diisocyanate polyaddition and final hardening occurs by means of radical polymerization.

2. The use according to claim 1, **characterized in that** as diisocyanate, hexamethylene diisocyanate (HDI), isophorone diisocyanate (IPDI) or bis-(4-isocyanatocyclohexyl) methane (H₁₂ MDI) in monomeric or in oligomeric form, e.g. in form of trimers is provided.

3. The use according to one of claims 1 or 2, **characterized in that** the material contains at least 5 to 60 wt.%, preferably 7.5 to 45 wt.%, particularly preferably 9 to 20 wt.% diisocyanate, referred to the total mass of the material.

4. The use according to one of claims 1 to 3, **characterized in that** for the material, polyols, namely compounds with at least three hydroxyl groups are provided as hydroxyl compound.

5. The use according to claim 4, **characterized in that** alcoholic OH groups are used as hydroxyl groups.

6. The use according to claim 5, **characterized in that** monomeric or polymeric aliphatic, alicylic or aromatic compounds with three to eight OH groups are used.

7. The use according to one of claims 1 to 3, **characterized in that** aliphatic and/or cycloaliphatic diols, in particular ethylene glycol, di- and tri-ethylene glycol, 1,2- and 1,3-propane diol, di- and tri-propylene glycol, 1,2- and 1,3- or 1,4-butane diol, 1,6-hexane diol and cyclohexane diol are used as hydroxyl groups.

8. The use according to one of claims 4 to 6, **characterized in that** polyesterpolyols, polyether-polyols and/or polyalcohols containing ether as well as ester groups are used as hydroxyl groups.

9. The use according to one of claims 1 to 8, **characterized in that** hydroxyl compounds having an equivalent weight from 50 to 1,000 per hydroxyl group are used.

10. The use according to one of claims 1 to 9, **characterized in that** hydroxyl compounds having a branched structure are used.

11. The use according to one of claims 1 to 10, **characterized in that** the material comprises, in addition to the hydroxyl groups, radically polymerizable groups such as vinyl, acryl and/or methacryl groups, preferably hydroxyethyl-(meth)-acrylate such as 2-hydroxyethyl acrylate, 2-hydroxyethyl methacrylate, 1,4-butanediol acrylate, 1,4-butanediol dimethacrylate, 2-hydroxypropyl acrylate, 2-hydroxypropyl methacrylate, 4-hydroxybutyl acrylate and 4-hydroxybutyl methacrylate, as well as mixtures thereof, in particular mixtures of monomeric and polymeric hydroxyl compounds.

12. The use according to claim 1 to 11, **characterized in that** the concentration of the hydroxyl compounds is in the range from 4 to 40 wt.% referred to the total mass of the material.

13. The use according to one of claims 1 to 12, **characterized in that** the material contains radically polymerizable monomers, in particular mono- and polyfunctional (meth)acrylates, such as 2,2-bis[4-(acryloyloxy ethoxy)phenyl]propane, 2,2-bis [4-(2-hydroxy-3-acryloyloxy propane)phenyl]propane, 1,3-butanediol di(meth)acrylate, 1,4-butane-diol di(meth)acrylate, 1,6-hexanediol di-(meth)-acrylate, mono-, di-, tri- and tetra-ethyleneglycol di(meth)acrylate, isobornyl acrylate, 2-10 fold ethoxylated bisphenol-A-di(meth)acrylates.

14. The use according to claim 13, **characterized in that** the material contains monomers, namely urethane di(meth)acrylates such as e.g. 7,7,9-trimethyl-4,13-dioxo-3,14-dioxa-5,12-diaza-hexadecane-1,16-diol-dimethacrylate or 7,9,9-trimethyl-4,13-dioxo-3,14-dioxa-5,12-diaza-hexadecane-1,16-diol-dimethacrylate.

15. The use according to one of claims 13 or 14, **characterized in that** the material contains monomers or monomer mixtures comprising at least one monomer with at least two radically polymerizable groups and thus acting as crosslinker.

16. The use according to one of claims 1 to 15, **characterized in that** the material contains filler materials, in particular inorganic glass, glass ceramic and ceramic powders, pyrogenic and precipitated silicic acids, zeolites, filled and unfilled organic filler materials based on poly(meth)acrylates or polycarbonates as well as mixtures of the mentioned filler materials.

17. The use according to claim 16, **characterized in that** the size of the filler materials is in the range from 10 nm to 200 µm, preferably in the range between 12 nm and 50 µm.

18. The use according to one of claims 1 to 17, **characterized in that** the material contains as initiators for the radical polymerization campher-quinone, 2,4,6-trimethylbenzoylphenyl phosphine oxide, bis(2,4,6-trimethylbenzoyl) phenylphosphine oxide, 2,4,6-trimethylbenzoyl diphenylphosphine oxide, 2-methyl-1,4-(methylthio) phenyl-2-morpholinopropane-1-on, bis(2,6-dimethoxybenzol)-2,4,4-trimethylpentyl phosphine oxide, 2-hydroxy-2-methyl-l-phenylpropane-l-on, 2-benzyl-2-dimethylamino-1-(4-morpholinophenyl)butanone-1, 2,2-di-methoxy-1,2-diphenylethane-1-on, 1-hydroxycyclo-hexylphenyl ketone, and mixtures thereof or also dibenzoyl peroxide.

19. The use according to claim 18, **characterized in that** the initiators are used in combination with synergists from the compound class of the tertiary amines such as e.g. N-methyl diethanolamine, dimethyl-p-toluidine, dihydroxyethyl-p-toluidine or triethanolamine.

20. The use according to one of claims 1 to 19, **characterized in that** the material contains stabilizers, in order to avoid a premature polymerization of the material, in particular compounds such as 2,6-di-tert.-butyl-p-cresol, hydroquinone, hydroquinone monomethyl ether, phenol and benzoquinone, 2,6-di-tert.-butyl-p-cresol being preferred.

21. The use according to one of claims 1 to 20, **characterized in that** the material contains catalytic converters for the diisocyanate polyaddition in form of tin organyls, in particular di-n-octyl tin carboxylate, dibutyl tin oxide, dioctyl tin oxide, dibutyl tin glycolate, dilaurate, diacetate, maleate, dibutyl tin maleinate ester, dilauryl tin diacetate, di-n-octyl tin maleinate ester, dibutyl tin alkyl mercaptide and dibutyl tin mercapto ester.

22. A method for producing noise protection parts, swimming protection parts, or otoplastics from a material according to one of claims 1 to 21, **characterized in that** the material is provided in two components, so that the reactive components of the material, namely the diisocyanate and the hydroxyl compound, are separate from each other, that the components are mixed, and the mixture is introduced into the ear or the auricle, and the material is pre-hardened there in a first step by the isocyanate polyaddition, then the blank is removed from the ear, if applicable is reworked, and then the final hardening takes place in a second step by radical polymerization.

23. The method according to claim 22, **characterized in that** the two components of the material are used in mixing ratios from 10:1 to 1:1, referred to the volume, preferably a bigger amount of hydroxyl compound.

24. The method according to one of claims 22 or 23, **characterized in that** the first component is provided in the form of a mixture of the hydroxyl compound, radically polymerizable monomer, filler materials, catalytic converters, stabilizers, synergists and if applicable additives, and the second component is provided in the form of a mixture of diisocyanate, filler material, stabilizers and if applicable additives.

## Revendications

1. Utilisation d'un matériau pour la production de pièces façonnées de protection contre le bruit, de pièces façonnées pour la pratique de la natation, d'éléments otoplastiques à insérer dans l'oreille et/ou dans le pavillon de l'oreille externe humaine et pour l'utilisation comme gel pour la fixation d'obturateurs à évents dans l'oreille, dans laquelle ce matériau consiste en au moins un diisocyanate et au moins un composé hydroxylé comprenant au moins deux groupes OH, de sorte qu'un pré-durcissement du matériau ait lieu au moyen de polyaddition de diisocyanate et un durcissement final ait lieu au moyen de polymérisation radicalique.

2. Utilisation selon la revendication 1, **caractérisée en ce que** hexaméthylène-diisocyanate (HDI), isophorone-diisocyanate (IPDI) ou bis-(4-isocyana-tocyclohexyle)-méthane (H₁₂ MDI) en forme monomérique ou oligomérique, p.ex. en forme de trimères est prévu comme diisocyanate.

3. Utilisation selon une des revendications 1 ou 2, **caractérisée en ce que** le matériau contient au moins 5 à 60 % en poids, de préférence 7,5 à 45 % en poids, de préférence particulière 9 à 20 % en poids de diisocyanate, par rapport à la masse totale du matériau.

4. Utilisation selon une des revendications 1 à 3, **caractérisée en ce que** des polyoles, c'est-à-dire des composés ayant au moins trois groupes hydroxylés sont prévus comme composés hydroxylés pour le matériau.

5. Utilisation selon la revendication 4, **caractérisée en ce que** des groupes OH alcooliques sont utilisés comme groupes hydroxylés.

6. Utilisation selon la revendication 5, **caractérisée en ce que** des composés aliphatiques, alicyliques ou aromatiques monomériques ou polymériques ayant trois à huit groupes OH sont utilisés.

7. Utilisation selon une des revendications 1 à 3, **caractérisée en ce que**, des diols aliphatiques et/ou cycloaliphatiques diols, en particulier éthylène-glycol, di- et tri-éthylène-glycol, 1,2-et 1,3-propane-diol, di- et tri-propylène-glycol, 1,2- et 1,3- ou 1,4-butane-diol, 1,6-hexane-diol et cyclohexane-diol sont utilisés comme groupes hydroxylés.

8. Utilisation selon une des revendications 4 à 6, **caractérisée en ce que** des polyester-polyoles, polyéther-polyoles et/ou polyalcools contenant des groupes éther aussi bien qu'ester sont utilisés comme des groupes hydroxylés.

9. Utilisation selon une des revendications 1 à 8, **caractérisée en ce que** des composés hydroxylés ayant un poids équivalent de 50 à 1000 par groupe hydroxylé sont utilisés.

10. Utilisation selon une des revendications 1 à 9, **caractérisée en ce que** des composés hydroxylés ayant une structure branchée sont utilisés.

11. Utilisation selon une des revendications 1 à 10, **caractérisée en ce que** le matériau comprend, en addition aux groupes hydroxylés, des groupes polymérisables radicaliquement comme p.ex. des groupes vinyliques, acryliques et/ou méthacryliques, de préférence hydroxy-éthyle-(méth)-acrylate comme p.ex. 2-hydroxy-éthyle-acrylate, 2-hydroxy-éthyle-méthacrylate, 1,4-butane-diol-acrylate, 1,4-butane-diol-diméthacrylate, 2-hydroxy-propyle-acrylate, 2-hydroxy-propyle-méthacrylate, 4-hydroxy-butyle-acrylate et 4-hydroxy-butyle-méthacrylate, aussi bien que des mélanges de ceux-ci, en particulier des mélanges de composés hydroxylés monomériques et polymériques.

12. Utilisation selon la revendication 1 à 11, **caractérisée en ce que** la concentration des composés hydroxylés est comprise dans la gamme entre 4 et 40 % en poids par rapport à la masse totale du matériau.

13. Utilisation selon une des revendications 1 à 12, **caractérisée en ce que** le matériau contient des monomères polymérisables radicaliquement, en particulier des (méth)acrylates mono- et polyfonctionnels, comme p.ex. 2,2-bis-[4-(acryloyloxy-éthoxy)phényle]propane, 2,2-bis-[4-(2-hydroxy-3-acryloyloxy-propane)phényle]propane, 1,3-butane-diol-di(méth)acrylate, 1,4-butanediol-di(méth)-acrylate, 1,6-hexanediol-di-(méth)acrylate, mono-, di-, tri- et tétra-éthylène-glycol-di(méth)acrylate, isobornyle-acrylate, bisphénol-A-di(méth)-acrylates éthoxylés 2-10 fois.

14. Utilisation selon la revendication 13, **caractérisée en ce que** le matériau contient des monomères, c'est-à-dire des uréthane-di(méth)acrylates comme p.ex. 7,7,9-triméthyle-4,13-dioxo-3,14-dioxa-5,12-diaza-hexadécane-1,16-diol-diméthacrylate ou 7,9,9-triméthyle-4,13-dioxo-3,14-dioxa-5,12-diaza-hexadécane-1,16-diol-diméthacrylate.

15. Utilisation selon une des revendications 13 ou 14, **caractérisée en ce que** le matériau contient des monomères ou des mélanges de monomères comprenant au moins un monomère avec au moins deux groupes polymérisables radicaliquement et ainsi agissant comme des agents réticulants.

16. Utilisation selon une des revendications 1 à 15, **caractérisée en ce que** le matériau contient des matériaux de remplissage, en particulier poudres de verre inorganique, céramique-verre et céramique, acides siliciques pyrogéniques et précipités, zéolites, matériaux de remplissage organiques remplis et non-remplis sur la base de poly(méth)acrylates ou polycarbonates aussi bien que des mélanges des matériaux de remplissage cités.

17. Utilisation selon la revendication 16, **caractérisée en ce que** la taille des matériaux de remplissage est comprise dans la gamme entre 10 nm et 200 µm, de préférence dans la gamme entre 12 nm et 50 µm.

18. Utilisation selon une des revendications 1 à 17, **caractérisée en ce que** le matériau contient, comme initiateurs pour la polymérisation radicalique, de la camphoroquinone, oxyde de 2,4,6-triméthyle-benzoyle-phényle-phosphine, oxyde de bis-(2,4,6-triméthyle-benzoyle)-phényle-phosphine, oxyde de 2,4,6-triméthyle-benzoyle-diphényle-phosphine, 2-méthyle-1,4-(méthylethio)phényle-2-morpholino-propane-1-on, oxyde de bis-(2,6-diméthoxy-benzol)-2,4,4-triméthyle-pentyle-phosphine, 2-hydroxy-2-méthyle-1-phényle-propane-1-on, 2-benzyle-2-diméthyleamino-l-(4-morpholino-phényle)butanone-1, 2,2-diméthoxy-1, 2-diphényle-éthane-1-on, 1-hydroxy-cyclo-hexyle-phényle-cétone, et mélanges de ceux-ci ou aussi peroxyde de dibenzoyle.

19. Utilisation selon la revendication 18, **caractérisée en ce que** les initiateurs sont utilisés en combinaison avec des synergistes de la classe des composés des amines tertiaires comme p.ex. N-méthyle-diéthanol-amine, diméthyle-p-toluidine, di-hydroxyéthyle-p-toluidine ou triéthanol-amine.

20. Utilisation selon une des revendications 1 à 19, **caractérisée en ce que** le matériau contient des stabilisateurs, afin d'éviter une polymérisation prématurée du matériau, en particulier des composés comme p.ex. 2,6-di-tert.-butyle-p-crésol, hydroquinone, hydroquinone-monométhyle-éther, phénol et benzoquinone, le 2,6-di-tert.-butyle-p-crésol étant préféré.

21. Utilisation selon une des revendications 1 à 20, **caractérisée en ce que** le matériau contient des catalyseurs pour la polyaddition de diisocyanate en forme d'organyles d'étain, en particulier di-n-octyle-carboxylate d'étain, dibutyle-oxyde d'étain, dioctyle-oxyde d'étain, dibutyle-glycolate d'étain, dilaurate, diacétate, maléate, dibutyle-maléinate-ester d'étain, dilauryle-diacétate d'étain, di-n-octyle-maléinate-ester d'étain, dibutyle-alkyle-mercaptide d'étain et dibutyle-mercapto-ester d'étain.

22. Procédé de production de pièces façonnées de protection contre le bruit, de pièces façonnées pour la pratique de la natation, ou d'éléments otoplastiques à partir d'un matériau selon une des revendications 1 à 21, **caractérisé en ce que** le matériau est prévu en deux composants, c'est-à-dire les composants réactifs du matériau, a savoir le diisocyanate et le composé hydroxylé, sont séparés l'un de l'autre, les composants sont mélangés, et le mélange est introduit dans l'oreille ou le pavillon d'oreille externe, et le matériau est pré-durci là-dedans en une première étape par la polyaddition d'isocyanate, puis la pièce brute est retirée de l'oreille, le cas échéant est finie, et puis le durcissement final a lieu en une deuxième étape par la polymérisation radicalique.

23. Procédé selon la revendication 22, **caractérisé en ce que** les deux composants du matériau sont utilisés en des rapports de mélange de 10:1 à 1:1, selon le volume, de préférence une quantité plus grande de composé hydroxylé.

24. Procédé selon une des revendications 22 ou 23, **caractérisé en ce que** le premier composant est prévu en forme d'un mélange de composé hydroxylé, monomère polymérisable radicaliquement, matériaux de remplissage, catalyseurs, stabilisateurs, synergistes et le cas échéant additifs, et le deuxième composant est prévu en forme d'un mélange de diisocyanate, matériau de remplissage, stabilisateurs et le cas échéant additifs.
